(19) Europäisches Patentamt
European Patent Office
Office européen des brevets

(11) EP 1 642 918 B1

(12) EUROPEAN PATENT SPECIFICATION

(45) Date of publication and mention of the grant of the patent:
**24.12.2014 Bulletin 2014/52**

(21) Application number: **04722658.4**

(22) Date of filing: **23.03.2004**

(51) Int Cl.:
***C08G 65/44*** *(2006.01)* ***C07C 43/295*** *(2006.01)*

(86) International application number:
**PCT/JP2004/003915**

(87) International publication number:
**WO 2005/003211 (13.01.2005 Gazette 2005/02)**

(54) **PROCESS FOR PRODUCTION OF BIFUNCTIONAL PHENYLENE ETHER OLIGOMERS**

VERFAHREN ZUR HERSTELLUNG VON BIFUNKTIONELLEN PHENYLENETHEROLIGOMEREN

PROCEDE POUR LA PRODUCTION D'OLIGOMERES DE PHENYLENE ETHER BIFONCTIONNELS

(84) Designated Contracting States:
**DE FR GB**

(30) Priority: **02.07.2003 JP 2003190369**

(43) Date of publication of application:
**05.04.2006 Bulletin 2006/14**

(73) Proprietor: **MITSUBISHI GAS CHEMICAL COMPANY, INC.**
**Tokyo 100-0005 (JP)**

(72) Inventors:
- **ISHII, Kenji,**
**c/o Tokyo Laboratory of Mitsubishi**
**Katsushika-ku,**
**Tokyo 125-0051 (JP)**
- **NORISUE, Yasumasa,c/o Tokyo Lab. of Mitsubishi**
**Katsushika-ku,**
**Tokyo 125-0051 (JP)**
- **YANAGIDA, Katsuhiko,**
**Tokyo Lab. of Mitsubishi**
**Katsushika-ku,**
**Tokyo 125-0051 (JP)**
- **MIYAMOTO, Makoto,**
**Tokyo Laboratory of Mitsubishi**
**Katsushika-ku,**
**Tokyo 125-0051 (JP)**
- **SHIMUTA, Masanori,**
**Tokyo Laboratory of Mitsubishi**
**Katsushika-ku,**
**Tokyo 125-0051 (JP)**
- **HIRAMATSU, Kiyonari,**
**Tokyo Lab. of Mitsubishi**
**Katsushika-ku,**
**Tokyo 125-0051 (JP)**

(74) Representative: **Srinivasan, Ravi Chandran**
**J A Kemp**
**14 South Square**
**Gray's Inn**
**London WC1R 5JJ (GB)**

(56) References cited:

| | |
|---|---|
| **EP-A- 0 215 257** | **WO-A1-03/076495** |
| **JP-A- 1 045 427** | **JP-A- 10 212 350** |
| **JP-A- 2003 012 796** | **JP-A- 2004 059 642** |
| **JP-A- 2004 115 619** | **US-A1- 2002 028 907** |
| **US-A1- 2003 130 438** | **US-A1- 2003 229 256** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 642 918 B1

## Description

Technical Field

The present invention relates to a process for the production of a bifunctional phenylene ether oligomer compound having phenolic hydroxyl groups at both terminals. It relates to a process for the production of a bifunctional phenylene ether oligomer compound which has no amine adduct and in addition has an extremely small remaining unreacted raw material phenol content.

Background Arts

Materials for use in electrical and electric equipment are required to have low dielectric characteristics for treating mass data at a high speed in the advanced information society and toughness for avoiding the occurrence of a micro crack due to thermal shock, etc. The use of engineering plastics, such as polyphenylene ether (to be sometimes referred to as "PPE" hereinafter), as a material for the above usage is proposed.

However, although PPE has an excellent high frequency property, it is known that PPE has the following problems. PPE is poor in the compatibility with thermosetting resins such as epoxy resins or cyanate resins. PPE is poor in molding processability since it has a high melt viscosity. Solvents which can dissolve PPE are limited to aromatic hydrocarbon solvents such as toluene, benzene and xylene and halogenated hydrocarbon solvents such as methylene chloride and chloroform, and therefore the workability of PPE is poor.

For improving the compatibility, a method which brings about an improvement by blending PPE with another resin as a compatibilizing agent and the pseudo-IPN structuralization (for example, JP-A-11-21452 publication (pp. 1 - 6)) of PPE and a cyanate resin have been studied. However, the molding workability and heat resistance have not yet been overcome. As means for improving the moldability, a method for converting a high-molecular PPE into a low-molecular PPE has been studied. For example, a method in which a high-molecular PPE and a bivalent phenol are redistributed in the presence of a radical catalyst (for example, JP-A-9-291148 (pp.1 - 3)) and a method in which a bivalent phenol and a monovalent phenol are oxidatively polymerized (for example, JP-B-8-011747 (pp.1 - 3)) are known. However, a polymer compound is present in each method so that it is impossible to efficiently obtain a bifunctional phenylene ether oligomer compound having a desired molecular weight.

Under the above circumstances, the present inventors have found an epoch-making means for efficiently producing a bifunctional phenylene ether oligomer compound having a desired average molecular weight by oxygen oxidation of bivalent and monovalent phenols in the presence of a catalyst and an amine (JP-A-2003-012796) . However, even in this method, unreacted raw material phenols are present when the supply of the raw material phenols is finished. In this case, it is possible to continue the oxidation polymerization until the unreacted phenols are consumed, i.e., to carry out a maturation reaction. However, when the maturation reaction is carried out for a long time, a product varies with the passage of time and decreases in quality. In particular, when the maturation reaction is continued after the raw material phenols are entirely reacted, the above tendency is remarkable. Further, uneconomically, the reaction time becomes longer.

Further, with regard to polyphenylene ether resins obtained by oxidation polymerization of phenols, it is widely known that an aliphatic secondary amine, which is used in the oxidation polymerization, is added to a benzylic position of an ortho position of a terminal phenol (for example, JP-A-52-897 (pp. 1 - 7)). There is a problem that when a terminal phenolic hydroxyl group of the polyphenylene ether resin is induced to another functional group, the above-mentioned added amine interrupts the reaction or decreases the stability of the functional group. As a method for decreasing the generation amount of an amine adduct, a method which uses a specific amine is proposed (for example, JP-A-62-131022 (pp. 1 - 4)). However the effect thereof is insufficient. Further, a method in which an amine added to the polyphenylene ether resin is substituted with an alcohol is proposed (for example, JP-A-5-148357 (pp. 1 - 5)), while a problem is that the number of production steps increases.

Therefore, it is an object of the present invention to provide a process for efficiently producing a bifunctional phenylene ether oligomer compound having no amine adduct which compound has the excellent electric characteristics and toughness of PPE, is improved in the compatibility with a thermosetting resin and in molding processability, is soluble in a general-purpose ketone solvent, has an extremely small unreacted phenol content and has an easily-modifiable terminal phenolic hydroxyl group.

KR 2003 0032860 provides a process for producing a bifunctional phenylene ether oligomer compound having a controlled average molecular weight, comprising carrying out oxidation polymerization by charging the reactor with a mixture containing a catalyst, an amine and a solvent in advance, and then dropwise adding a mixture of a bivalent phenol, a monovalent phenol and an amine.

KR 2003 0003107 provides a bifunctional phenylene ether oligomer obtained by oxidation polymerization of a bivalent phenol and a monovalent phenol, using a variety of amines as catalyst in the reaction.

Disclosure of the Invention

The present invention provides a process for the production of a bifunctional phenylene ether oligomer compound having no amine adduct, represented by the formula (1), which process comprises oxidatively polymerizing a bivalent phenol of the formula (2) and a monovalent phenol of the formula (3) in the presence of (a) a copper-containing catalyst, and (b) an amine co-catalyst mixture consisting essentially of a tertiary amine and a secondary amine having a secondary alkyl group, a tertiary alkyl group or an aryl group, wherein 20% to 70% of the amine (b), based on the total amount thereof, is charged into a reactor in advance and the balance of 30 to 80% is added subsequently.

[Chemical formula 1]

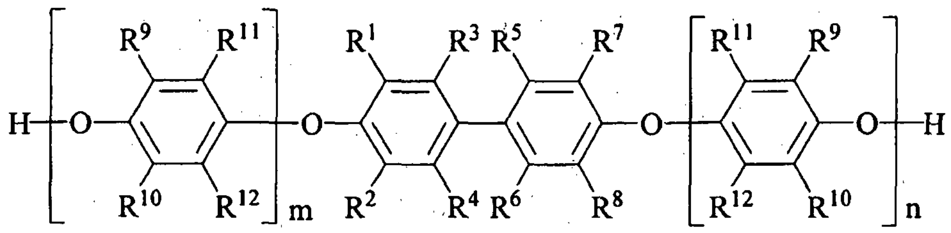

(1)

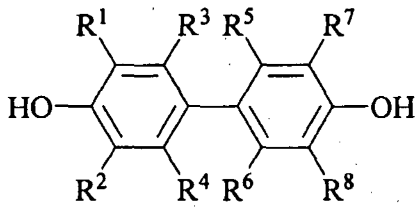

(2)

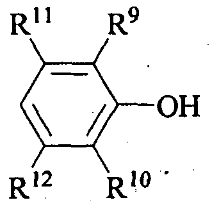

(3)

wherein $R^1$, $R^2$, $R^3$, $R^7$, $R^8$, $R^9$ and $R^{10}$ are the same or different and represent a halogen atom, an alkyl group having 6 or less carbon atoms or a phenyl group, $R^4$, $R^5$, $R^6$, $R^{11}$ and $R^{12}$ are the same or different and represent a hydrogen atom, a halogen atom, an alkyl group having 6 or less carbon atoms or a phenyl group, and each of m and n is an integer of from 0 to 25, provided that at least one of a and b is not 0.

The process of the present invention stably and efficiently produces in high quality a bifunctional phenylene ether oligomer compound of the formula (1) having small amounts of remaining unreacted phenols by charging 20 % to 70 %, based on the total amount to be used, of the mixture consisting essentially of the tertiary amine and the secondary amine having a secondary alkyl group, a tertiary alkyl group or an aryl group into a reactor in advance, and adding the balance of from 30 to 80 % with the advance of the reaction.

Most Preferred Embodiment of the Invention

The bivalent phenol used in the present invention refers to a bivalent phenol represented by the formula (2).

[Chemical formula 2]

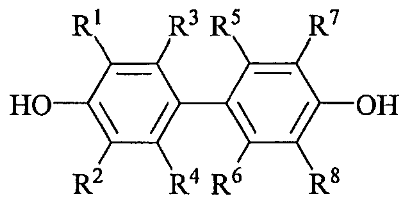

(2)

In the bivalent phenol of the formula (2), $R^1$, $R^2$, $R^3$, $R^7$ and $R^8$ are the same or different and represent a halogen atom, an alkyl group having 6 or less carbon atoms or a phenyl group and $R^4$, $R^5$ and $R^6$ are the same or different and represent a hydrogen atom, a halogen atom, an alkyl group having 6 or less carbon atoms or a phenyl group. In the bivalent phenol of the formula (2), it is essentially required that $R^1$, $R^2$, $R^3$, $R^7$ and $R^8$ are not hydrogen atoms. Specifically, 2,3,3',5,5'-pentamethyl-(1,1'-biphenyl)-4,4'-diol and 2,2'3,3',5,5'-hexamethyl-(1,1'-biphenyl)-4,4'-diol are preferred.

The monovalent phenol used in the present invention refers to a monovalent phenol represented by the formula (3).

[Chemical formula 3]

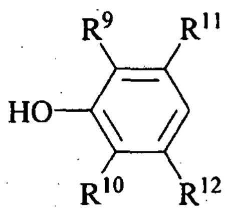

(3)

In the formula (3), $R^9$ and $R^{10}$ are the same or different and represent a halogen atom, an alkyl group having 6 or less carbon atoms or a phenyl group and $R^{11}$ and $R^{12}$ are the same or different and represent a hydrogen atom, a halogen atom, an alkyl group having 6 or less carbon atoms or a phenyl group. In particular, it is preferred to use a monovalent phenol of the formula (3) having substituents at 2- and 6-positions alone or use this phenol in combination with a monovalent phenol of the formula (3) having substituents at 2-, 3- and 6-positions or at 2-, 3-, 5- and 6-positions. Further, when used alone, 2, 6-dimethylphenol is preferred. When used in combination, it is preferred to use 2,6-dimethylphenol and 2,3,6-trimethylphenol.

The bifunctional phenylene ether oligomer compound represented by the formula (1) of the present invention is obtained by oxidatively polymerizing the bivalent phenol of the formula (2) and the monovalent phenol of the formula (3). Preferably, the molar ratio of the bivalent phenol of formula (2) and the monovalent phenol of formula (3) is 1:1 to 1:15. The oxidation method is typically a method which uses an oxygen gas or air directly. Further, an electrode oxidation method is also adaptable. The oxidation method is not specially limited. Air oxidation is preferable in view of economical plant and equipment investment, while it is more preferable in view of safety to carry out the oxidation polymerization with controlling the oxygen concentration in a reactor at the limit oxygen concentration of explosion limit or lower. As a method of the oxidation polymerization at the limit oxygen concentration or lower, there are a method in which the oxidation polymerization is carried out with air while an inert gas is supplied into a gaseous phase and a method in which the oxidation polymerization is carried out with a mixture gas obtained by mixing an inert gas and air and having an oxygen concentration of 3 to 15 %. For carrying out the oxidation polymerization, a pressure of from atmospheric pressure to 20 $kg/cm^2$ is generally selected.

The catalyst used for the oxidation polymerization is a copper containing catalyst and includes copper salts such as CuCl, CuBr, $Cu_2SO_4$, $CuCl_2$, $CuBr_2$, $CuSO_4$ and CuI. These copper salts may be used alone or in combination. The catalyst is not specially limited to these copper salts. In addition to the above catalyst, an amine is used. The amine includes a secondary amine such as diisopropylamine, di-sec-butylamine, di-t-butylamine, di-t-amylamine, dicyclopentylamine, dicyclohexylamine, diphenylamine, p,p'-ditolylamine, m,m'-ditolylamine, ethyl-t-butylamine, N,N'-di-t-butylethylenediamine, methylcyclohexylamine and methylphenylamine, and a tertiary amine such as triethylamine, methyldiethylamine, n-butyldimethylamine, benzyldimethylamine, phenyldimethylamine, N,N-dimethyl-p-toluidine triphenylamine, N,N'-dimethylpiperazine and 2,6-dimethylpyridine. The amine is not specially limited to these amines so long as it is a combination of a tertiary amine and a secondary amine having a secondary alkyl group, a tertiary alkyl group or an aryl group. These amines are a co-catalyst for the copper-containing catalyst. The amount thereof is preferably

0.1 mol to 50 mol per 1 mol of the copper-containing catalyst. The use of the above amine can give a bifunctional phenylene ether oligomer compound having no amine adduct. The functional group conversion of the above bifunctional phenylene ether oligomer compound having no amine adduct is not interrupted by an added amine and therefore its phenolic hydroxyl group can be easily and efficiently converted into a different functional group.

The present invention can stably produce in high quality a bifunctional phenylene ether oligomer compound of the formula (1) having a small remaining unreacted raw material phenol content by charging the mixture of the tertiary amine and the secondary amine having a secondary alkyl group, a tertiary alkyl group or an aryl group, in an amount of 20 to 70 %, based on the total amount thereof to be used, in a reactor in advance and adding the balance of 30 to 80 % with the advance of the reaction. In this case, the copper-containing catalyst may be added dividedly or in one lump. It is preferable to add the copper-containing catalyst dividedly. Preferably, 20 to 100% of the catalyst, based on the total amount thereof, is charged in the reactor in advance and the balance of 0 to 80% is charged subsequently. In this case, the amount of unreacted raw material phenols is further decreased. When the entire amount of the amine is added in a reactor from the beginning, catalytic activity is sufficient in the initial stage of supply of the raw materials, while the catalyst activity gradually decreases with the advance of the raw material supply and the amount of unreacted raw material phenols increases. These problems has been overcome by dividing the amine in advance, charging 20 to 70 %, preferably 30 to 65 %, based on the total amount to be used, of the amine in a reactor and supplying the balance with the advance of the reaction, according to the present invention.

In the present invention, a bifunctional phenylene ether oligomer compound of the formula (1) having a desired number average molecular weight can be efficiently produced by carrying out the reaction with supplying the bivalent phenol of the formula (2) and the monovalent phenol of the formula (3) in a specific molar ratio. For example, when 2, 2' 3, 3' 5, 5'-hexamethyl-(1,1'-biphenyl)-4,4'-diol as the bivalent phenol and 2, 6-dimethylphenol as the monovalent phenol are used in a molar ratio of 1: 3, a bifunctional phenylene ether oligomer compound having a number average molecular weight of 600 to 700 is obtained. When the molar ratio is 1:5, a bifunctional phenylene ether oligomer compound having a number average molecular weight of 850 to 950 is obtained. When the molar ratio is 1:10, a bifunctional phenylene ether oligomer compound having a number average molecular weight of 1,450 to 1,550 is obtained.

In the present invention, an activator for a gas-liquid interface such as a surface active agent or a phase transfer catalyst may be used as required. Examples of the surface active agent include a nonionic surface active agent, a cationic surface active agent, an anionic surface active agent and an ampholytic surface active agent. Examples of the nonionic surface active agent include polyoxy alkyl ethers such as polyoxyethylene decyl ether, polyoxyethylene dodecyl ether, polyoxyethylene cocoalkyl ether, polyoxypropylene decyl ether, polyoxypropylene dodecyl ether and polyoxypropylene cocoalkyl ether; polyoxy alkylene aromatic substituted alkyl ethers such as polyoxyethylene octyl phenyl ether, polyoxyethylene nonyl phenyl ether, polyoxypropylene octyl phenyl ether and polyoxypropylene nonyl phenyl ether; higher alcohols such as decyl alcohol, dodecyl alcohol and tetradecyl alcohol; esters of polyoxy alkylene glycol and higher fatty acid such as polyoxyethylene laurate, polyoxyethylene palmitate and polyoxyethylene stearate; esters of polyhydric alcohol and higher fatty acid such as sorbitan sesquioleate, sorbitan monooleate, sorbitan monostearate, polyoxyethylene sorbitan monooleate and polyoxyethylene sorbitan monostearate; and diesters of higher fatty acid such as ethylene glycol distearate and polyoxyethylene distearate. Examples of the cationic surface active agent include lauryl trimethyl ammonium chloride, stearyl trimethyl ammonium chloride, distearyl dimethyl ammonium chloride and cationized cellulose. Examples of the anionic surface active agent include sodium lauryl sulfate, sodium polyoxyethylene lauryl sulfate, polyoxyethylene lauryl ether acetic acid, sodium polyoxyethylene lauryl ether acetate, lauric acid, myristic acid, palmitic acid, stearic acid and linoleic acid. Examples of the ampholytic surface active agent include lauryl dimethyl aminoacetic acid betaine, stearyl dimethyl aminoacetic acid betaine, lauryl dimethyl amine oxide, lauric acid amide propyl betaine and lauryl hydroxy sulfobetaine.

Examples of the phase transfer catalyst include tetramethyl ammonium chloride, tetramethyl ammonium bromide, tetramethyl ammonium acetate, tetra-n-butyl ammonium chloride, tetra-n-butyl ammonium bromide, tetra-n-butyl ammonium iodide, tetra-n-butyl ammonium fluoride, tetra-n-butyl ammonium borohydride, tetra-n-butyl ammonium tetrafluoroborate, trimethyl-n-octyl ammonium chloride, trimethylbenzyl ammonium chloride, triethyl-n-octyl ammonium chloride, triethylbenzyl ammonium chloride, tri-n-octyl ammonium chloride, tri-n-octyl ammonium bromide, methyltriphenyl ammonium chloride, methyltriphenyl ammonium bromide, tri-n-butyl-n-octyl ammonium chloride, tri-n-butyl benzyl ammonium chloride and tri-n-butyl benzyl ammonium bromide.

These surface active agents and phase transfer catalysts may be used alone or in combination. They are preferably used in amount of 0.1 to 20 mmol per 1 mol of the raw material phenols.

In the present invention, it is possible to continue the oxidation polymerization after the termination of the supply of the raw material phenols, as long as the unreacted phenols remain. However, from the aforementioned reasons, it is not preferred to continue the oxidation polymerization after all of the raw material phenols have been reacted, since it causes a decrease in quality and uneconomically lengthens the reaction time. According to the present invention, the bifunctional phenylene ether oligomer compound having an extremely small unreacted raw material phenol content can be obtained at the time of termination of the raw material phenol supply.

Then, a solvent used in the present invention is explained. A ketone solvent and an alcohol solvent have been thought to be a poor solvent in oxidation polymerization and their use is limited in a conventional PPE oxidation polymerization. However, the ketone solvent and the alcohol solvent can be used in the present invention. In conventional oxidation polymerizations, a ketone or an alcohol is not able to be used as a reaction solvent since a polymer which is not easily dissolved in an organic solvent generates. However, the product of the present invention is easily dissolved in a ketone or an alcohol so that the range of usable solvents widens largely. The ketone solvent or alcohol solvent may be used alone or in combination with a conventional solvent such as an aromatic hydrocarbon solvent typified by toluene, benzene or xylene, or a halogenated hydrocarbon solvent typified by methylene chloride or chloroform. The ketone solvent includes acetone, methyl ethyl ketone, diethyl ketone, methyl butyl ketone, methyl isobutyl ketone, etc. The alcohol solvent includes methanol, ethanol, propanol, isopropanol, butanol, ethylene glycol, propylene glycol, etc. The ketone solvent and the alcohol solvent are not limited to these examples.

The reaction temperature in the production process of the present invention is not specially limited so long as it does not enter the explosion limit of solvent used. It is preferably 30 to 50 °C. Since the oxidation polymerization is an exothermic reaction, it is difficult to control the temperature at 50 °C or higher and it is difficult to control a molecular weight. When it is 30 °C or lower, the temperature enters the range of explosion limit in some cases depending upon the solvent used so that safe production is impossible.

[Examples]

Then, the present invention will be concretely explained with reference to Examples and Comparative Examples, while the present invention shall not be specially limited to these Examples. A number average molecular weight (Mn), a weight average molecular weight (Mw) and the amount of unreacted phenols were measured according to the gel permeation chromatography (GPC) method. Data processing was carried out according to the GPC curve and molecular weight calibration curve of a sample. The molecular weight calibration curve was obtained by making an approximation of a relation between the molecular weight of a standard polystylene and the dissolution time thereof with the following equation,

$$LogM = A_0X^3 + A_1X^2 + A_2X + A_3 + A_4/X^2$$

wherein M : a molecular weight, X : an elution time - 19 (minute), and A : a coefficient.

A hydroxyl group equivalent was determined from an absorption intensity at 3, 600 $cm^{-1}$ in an IR analysis (solution cell method; cell thickness = 1mm) using 2, 6-dimethylphenol as a standard reference material.

(Example 1)

A longitudinally long reactor having a volume of 12 liters and equipped with a stirrer, a thermometer, an air-introducing tube and baffleplates was charged with 0.98 g (4.4 mmol) of $CuBr_2$, 0.32 g (1.9 mmol) of N,N'-di-t-butylethylenediamine, 9.78 g (96.6 mmol) of n-butyldimethylamine, 0. 6 g (2. 6 mmol) of sodium lauryl sulfate and 2, 000 g of toluene. The components were stirred at a reaction temperature of 40 °C. A mixed solution (bivalent phenol of the formula (2) : monovalent phenol of the formula (3) in molar ratio = 1 : 5) was obtained by dissolving 129.8 g (0.48 mol) of 2, 2' , 3, 3' , 5, 5' -hexamethyl-(1,1'-biphenyl)-4,4'-diol (to be referred to as "HMBP" hereinafter), 293.2 g (2.40 mol) of 2,6-dimethylphenol, 2.92 g (13.1 mmol) of $CuBr_2$, 0.96 g (5.6 mmol) of N,N'-di-t-butylethylenediamine and 29.32 g (289.8 mmol) of n-butyldimethylamine in 2, 200 g of methanol in advance. The mixed solution was dropwise added to the mixture in the reactor over 230 minutes while carrying out bubbling with 5.2L/min of a nitrogen-air mixed gas having an oxygen concentration of 8 %, and stirring was carried out. After the completion of the addition, 1,500 g of water in which 34.09 g (75.4 mmol) of tetrasodium ethylenediamine tetraacetate tetrahydrate was dissolved was added to the stirred mixture to terminate the reaction. An aqueous layer and an organic layer were separated. Then, the organic layer was washed with 1.0 N hydrochloric acid aqueous solution and then with pure water. The thus obtained solution was concentrated with an evaporator and then dried under a reduced pressure, to obtain 413.2 g of a phenylene ether oligomer compound. The phenylene ether oligomer compound had Mn of 900, Mw of 1,420, Mw/Mn of 1.58 and a hydroxyl group equivalent of 455. Unreacted HMBP was 1.5 % and unreacted 2,6-dimethylphenol was 0.1 %. No peak corresponding to amine was detected in its $^1$H-NMR measurement and accordingly it was confirmed that no amine adduct generated.

(Example 2)

A longitudinally long reactor having a volume of 12 liters and equipped with a stirrer, a thermometer, an air-introducing tube and baffleplates was charged with 0.78 g (3.5 mmol) of $CuBr_2$, 0.26 g (1.5 mmol) of N,N'-di-t-butylethylenediamine, 7.82 g (77.3 mmol) of n-butyldimethylamine, 0.3 g (0.8 mmol) of tri-n-octylmethyl ammonium chloride and 2, 000 g of toluene. The components were stirred at a reaction temperature of 40 °C. A mixed solution (bivalent phenol of the formula (2) : monovalent phenol of the formula (3) in molar ratio = 1 : 2) was obtained by dissolving 112.2 g (0.46 mol) of HMBP, 103.8 g (0.85 mol) of 2,6-dimethylphenol, 0.38 g (2.2 mmol) of N,N'-di-t-butylethylenediamine and 11.73 g (115.9 mmol) of n-butyldimethylamine in 2,000 g of methanol in advance. The mixed solution was dropwise added to the mixture in the reactor over 230 minutes while carrying out bubbling with 5.2L/min of a nitrogen-air mixed gas having an oxygen concentration of 8 %, and stirring was carried out. After the completion of the addition, 1,500 g of water in which 9.92 g (22.0 mmol) of tetrasodium ethylenediamine tetraacetate tetrahydrate was dissolved was added to the stirred mixture to terminate the reaction. An aqueous layer and an organic layer were separated. Then, the organic layer was washed with 1.0 N hydrochloric acid aqueous solution and then with pure water. The thus obtained solution was concentrated with an evaporator and then dried under a reduced pressure, to obtain 212.8 g of a bifunctional phenylene ether oligomer compound. The bifunctional phenylene ether oligomer compound had Mn of 550, Mw of 850, Mw/Mn of 1.55 and a hydroxyl group equivalent of 290. Unreacted HMBP was 1.3 % and unreacted 2, 6-dimethylphenol was less than 0.1 %. No peak corresponding to amine was detected in its $^1$H-NMR measurement and accordingly it was confirmed that no amine adduct generated.

(Example 3)

A longitudinally long reactor having a volume of 12 liters and equipped with a stirrer, a thermometer, an air-introducing tube and baffleplates was charged with 0.90 g (4.0 mmol) of $CuBr_2$, 0.29 g (1.7 mmol) of N,N'-di-t-butylethylenediamine, 9.0 g (88.9 mmol) of n-butyldimethylamine, 0.6 g (2. 6 mmol) of sodium lauryl sulfate and 2, 000 g of toluene. The components were stirred at a reaction temperature of 40 °C. A mixed solution (bivalent phenol of the formula (2) : monovalent phenol of the formula (3) in molar ratio = 1 : 2) was obtained by dissolving 129.8 g (0.48 mol) of HMBP, 120.1 g (0.98 mol) of 2, 6-dimethylphenol, 1.35 g (6.0 mmol) of $CuBr_2$, 0.44 g (2.6 mmol) of N,N'-di-t-butylethylenediamine and 13.57 g (134.1 mmol) of n-butyldimethylamine in 2,200 g of methanol in advance. The mixed solution was dropwise added to the mixture in the reactor over 230 minutes while carrying out bubbling with 5.2L/min of a nitrogen-air mixed gas having an oxygen concentration of 8 %, and stirring was carried out. After the completion of the addition, 1, 500 g of water in which 19.84 g (43.9 mmol) of tetrasodium ethylenediamine tetraacetate tetrahydrate was dissolved was added to the stirred mixture to terminate the reaction. An aqueous layer and an organic layer were separated. Then, the organic layer was washed with 1.0 N hydrochloric acid aqueous solution and then with pure water. The thus obtained solution was concentrated with an evaporator and then dried under a reduced pressure, to obtain 245.7 g of a bifunctional phenylene ether oligomer compound. The bifunctional phenylene ether oligomer compound had Mn of 560, Mw of 860, Mw/Mn of 1.54 and a hydroxyl group equivalent of 290. Unreacted HMBP was 1.1 % and unreacted 2, 6-dimethylphenol was less than 0.1 %. No peak corresponding to amine was detected in its $^1$H-NMR measurement and accordingly it was confirmed that no amine adduct generated.

(Example 4)

A longitudinally long reactor having a volume of 12 liters and equipped with a stirrer, a thermometer, an air-introducing tube and baffleplates was charged with 1.95 g (8.7 mmol) of $CuBr_2$, 0.64 g (3.7 mmol) of N,N'-di-t-butylethylenediamine, 19.55 g (193.2 mmol) of n-butyldimethylamine, 0.7 g (1.7 mmol) of tri-n-octylmethyl ammonium chloride and 2,000 g of toluene. The components were stirred at a reaction temperature of 40 °C. A mixed solution (bivalent phenol of the formula (2) : monovalent phenol of the formula (3) in molar ratio = 1 : 5) was obtained by dissolving 129.8 g (0.48 mol) of HMBP, 293.2 g (2.40 mol) of 2, 6-dimethylphenol, 1.95 g (8.7 mmol) of $CuBr_2$, 0.64 g (3.7 mmol) of N,N'-di-t-butylethylenediamine and 19.55 g (193.2 mmol) of n-butyldimethylamine in 2,200 g of methanol in advance. The mixed solution was dropwise added to the mixture in the reactor over 230 minutes while carrying out bubbling with 5.2L/min of a nitrogen-air mixed gas having an oxygen concentration of 8 %, and stirring was carried out. After the completion of the addition, 1,500 g of water in which 34.09 g (75.4 mmol) of tetrasodium ethylenediamine tetraacetate tetrahydrate was dissolved was added to the stirred mixture to terminate the reaction. An aqueous layer and an organic layer were separated. Then, the organic layer was washed with 1.0 N hydrochloric acid aqueous solution and then with pure water. The thus obtained solution was concentrated with an evaporator and then dried under a reduced pressure, to obtain 416. 9 g of a phenylene ether oligomer compound. The phenylene ether oligomer compound had Mn of 930, Mw of 1,470, Mw/Mn of 1.58 and a hydroxyl group equivalent of 470. Unreacted HMBP was 0.5 % and unreacted 2, 6-dimethylphenol was less than 0.1 %. No peak corresponding to amine was detected in its $^1$H-NMR measurement and accordingly it was confirmed that no amine adduct generated.

(Example 5)

A longitudinally long reactor having a volume of 12 liters and equipped with a stirrer, a thermometer, an air-introducing tube and baffleplates was charged with 2.34 g (10.5 mmol) of $CuBr_2$, 0.76 g (4.4 mmol) of N,N'-di-t-butylethylenediamine, 23.46 g (231.8 mmol) of n-butyldimethylamine, 0.9 g (2.2 mmol) of tri-n-octylmethyl ammonium chloride and 2, 000 g of toluene. The components were stirred at a reaction temperature of 40 °C. A mixed solution (bivalent phenol of the formula (2) : monovalent phenol of the formula (3) in molar ratio = 1 : 10) was obtained by dissolving 75.70 g (0.28 mol) of HMBP, 342.1 g (2.80 mol) of 2, 6-dimethylphenol, 1.56 g (7.0 mmol) of $CuBr_2$, 0.51 g (3.0 mmol) of N,N'-di-t-butylethylenediamine and 15.64 g (154.6 mmol) of n-butyldimethylamine in 1,500 g of methanol in advance. The mixed solution was dropwise added to the mixture in the reactor over 230 minutes while carrying out bubbling with 5.2L/min of a nitrogen-air mixed gas having an oxygen concentration of 8 %, and stirring was carried out. After the completion of the addition, 1,500 g of water in which 19.84 g (43.9 mmol) of tetrasodium ethylenediamine tetraacetate tetrahydrate was dissolved was added to the stirred mixture to terminate the reaction. An aqueous layer and an organic layer were separated. Then, the organic layer was washed with 1.0 N hydrochloric acid aqueous solution and then with pure water. The thus obtained solution was concentrated with an evaporator and then dried under a reduced pressure, to obtain 408.4 g of a phenylene ether oligomer compound. The phenylene ether oligomer compound had Mn of 1,490, Mw of 2,370, Mw/Mn of 1.59 and a hydroxyl group equivalent of 760. Unreacted HMBP was 0.2 % and unreacted 2,6-dimethylphenol was 0.2 %. No peak corresponding to amine was detected in its $^1$H-NMR measurement and accordingly it was confirmed that no amine adduct generated.

(Example 6)

A longitudinally long reactor having a volume of 12 liters and equipped with a stirrer, a thermometer, an air-introducing tube and baffleplates was charged with 1.95 g (8.7 mmol) of $CuBr_2$, 0.64 g (3.7 mmol) of N,N'-di-t-butylethylenediamine, 19.55 g (193.2 mmol) of n-butyldimethylamine, 0.6 g (1.9 mmol) of tetra-n-butyl ammonium bromide and 2,000 g of methyl ethyl ketone. The components were stirred at a reaction temperature of 40 °C. A mixed solution (bivalent phenol of the formula (2) : monovalent phenol of the formula (3) in molar ratio = 1 : 5) was obtained by dissolving 129.8 g (0.48 mol) of HMBP, 293.2 g (2.40 mol) of 2, 6-dimethylphenol, 1.95 g (8.7 mmol) of $CuBr_2$, 0.64 g (3.7 mmol) of N,N'-di-t-butylethylenediamine and 19.55 g (193.2 mmol) of n-butyldimethylamine in 1,500 g of methyl ethyl ketone in advance. The mixed solution was dropwise added to the mixture in the reactor over 230 minutes while carrying out bubbling with 5.2L/min of a nitrogen-air mixed gas having an oxygen concentration of 8 %, and stirring was carried out. After the completion of the addition, 1,500 g of water in which 19.84 g (43.9 mmol) of tetrasodium ethylenediamine tetraacetate tetrahydrate was dissolved was added to the stirred mixture to terminate the reaction. An aqueous layer and an organic layer were separated. Then, the organic layer was washed with 1.0 N hydrochloric acid aqueous solution and then with pure water. The thus obtained solution was concentrated with an evaporator and then dried under a reduced pressure, to obtain 415.3 g of a phenylene ether oligomer compound. The phenylene ether oligomer compound had Mn of 920, Mw of 1, 440, Mw/Mn of 1.57 and a hydroxyl group equivalent of 465. Unreacted HMBP was 0.9 % and unreacted 2,6-dimethylphenol was 0.1 %. No peak corresponding to amine was detected in its $^1$H-NMR measurement and accordingly it was confirmed that no amine adduct generated.

(Example 7)

A longitudinally long reactor having a volume of 12 liters and equipped with a stirrer, a thermometer, an air-introducing tube and baffleplates was charged with 1.95 g (8.7 mmol) of $CuBr_2$, 0.64 g (3.7 mmol) of N,N'-di-t-butylethylenediamine, 19.55 g (193.2 mmol) of n-butyldimethylamine, 0.6 g (1.9 mmol) of tetra-n-butyl ammonium bromide and 2,000 g of toluene. The components were stirred at a reaction temperature of 40 °C. A mixed solution (bivalent phenol of the formula (2) : monovalent phenol of the formula (3) in molar ratio = 1 : 5) was obtained by dissolving 129.8 g (0.48 mol) of HMBP, 293.2 g (2.40 mol) of 2, 6-dimethylphenol, 1.95 g (8.7 mmol) of $CuBr_2$, 0.64 g (3.7 mmol) of N,N'-di-t-butylethylenediamine and 19.55 g (193.2 mmol) of n-butyldimethylamine in 2,200 g of methanol in advance. The mixed solution was dropwise added to the mixture in the reactor over 95 minutes while carrying out bubbling with 3.5L/min of air, and stirring was carried out. During this addition, 3.5 L/min of a nitrogen gas was flown in gaseous phase. After the completion of the addition, 1,500 g of water in which 19.84 g (43.9 mmol) of tetrasodium ethylenediamine tetraacetate tetrahydrate was dissolved was added to the stirred mixture to terminate the reaction. An aqueous layer and an organic layer were separated. Then, the organic layer was washed with 1.0 N hydrochloric acid aqueous solution and then with pure water. The thus obtained solution was concentrated with an evaporator and then dried under a reduced pressure, to obtain 414.4 g of a phenylene ether oligomer compound. The phenylene ether oligomer compound had Mn of 920, Mw of 1,460, Mw/Mn of 1.59 and a hydroxyl group equivalent of 465. Unreacted HMBP was 0.8 % and unreacted 2, 6-dimethylphenol was less than 0.1 %. No peak corresponding to amine was detected in its $^1$H-NMR measurement and

accordingly it was confirmed that no amine adduct generated.

(Example 8)

A longitudinally long reactor having a volume of 12 liters and equipped with a stirrer, a thermometer, an air-introducing tube and baffleplates was charged with 2.14 g (9.6 mmol) of $CuBr_2$, 0.76 g (4.4 mmol) of N,N'-di-t-butylethylenediamine, 15.64 g (154.6 mmol) of n-butyldimethylamine, 0.6 g (1.5 mmol) of tri-n-octylmethyl ammonium chloride and 2,000 g of toluene. The components were stirred at a reaction temperature of 40 °C. A mixed solution (bivalent phenol of the formula (2) : monovalent phenol of the formula (3) in molar ratio = 1 : 10) was obtained by dissolving 75.70 g (0.28 mol) of HMBP, 342.1 g (2.80 mol) of 2, 6-dimethylphenol, 1.76 g (7.9 mmol) of $CuBr_2$, 0.51 g (3.0 mmol) of N,N'-di-t-butylethylenediamine and 23.46 g (231.8 mmol) of n-butyldimethylamine in 1,500 g of methanol in advance. The mixed solution was dropwise added to the mixture in the reactor over 230 minutes while carrying out bubbling with 5.2L/min of a nitrogen-air mixed gas having an oxygen concentration of 8 %, and stirring was carried out. After the completion of the addition, 1,500 g of water in which 19.84 g (43.9 mmol) of tetrasodium ethylenediamine tetraacetate tetrahydrate was dissolved was added to the stirred mixture to terminate the reaction. An aqueous layer and an organic layer were separated. Then, the organic layer was washed with 1.0 N hydrochloric acid aqueous solution and then with pure water. The thus obtained solution was concentrated with an evaporator and then dried under a reduced pressure, to obtain 410.1 g of a phenylene ether oligomer compound. The phenylene ether oligomer compound had Mn of 1,490, Mw of 2,380, Mw/Mn of 1.60 and a hydroxyl group equivalent of 755. Unreacted HMBP was 0.3 % and unreacted 2,6-dimethylphenol was 0.1 %. No peak corresponding to amine was detected in its $^1H$-NMR measurement and accordingly it was confirmed that no amine adduct generated.

(Example 9)

Example 4 was repeated except that 3.90 g (17.5 mmol) of $CuBr_2$ was charged in the longitudinally long reactor in one lump in place of the divided additions of $CuBr_2$.

The amount of the thus obtained bifunctional phenylene ether oligomer compound was 408.2 g. The bifunctional phenylene ether oligomer compound had Mn of 860, Mw of 1,330, Mw/Mn of 1.55 and a hydroxyl group equivalent of 435. Unreacted HMBP was 4.3 % and unreacted 2,6-dimethylphenol was 0.9 %. No peak corresponding to amine was detected in its $^1H$-NMR measurement and accordingly it was confirmed that no amine adduct generated.

(Example 10)

Example 5 was repeated except that 3.90 g (17.5 mmol) of $CuBr_2$ was charged in the longitudinally long reactor in one lump in place of the divided additions of $CuBr_2$.

The amount of the thus obtained bifunctional phenylene ether oligomer compound was 401.0 g. The bifunctional phenylene ether oligomer compound had Mn of 1,450, Mw of 2,330, Mw/Mn of 1.61 and a hydroxyl group equivalent of 730. Unreacted HMBP was 3.9 % and unreacted 2,6-dimethylphenol was 0.7%. No peak corresponding to amine was detected in its $^1H$-NMR measurement and accordingly it was confirmed that no amine adduct generated.

(Comparative Example 2)

A longitudinally long reactor having a volume of 12 liters and equipped with a stirrer, a thermometer, an air-introducing tube and baffleplates was charged with 10.85 g (48.8 mmol) of $CuBr_2$, 286.83 g (2.22 mmol) of di-n-butylamine and 2, 000 g of toluene. The components were stirred at a reaction temperature of 40 °C. A mixed solution (bivalent phenol of the formula (2) : monovalent phenol of the formula (3) in molar ratio = 1 : 5) was obtained by dissolving 129.32 g (0.48 mol) of HMBP, 292.19 g (2.40 mol) of 2, 6-dimethylphenol in 2, 200 g of methanol in advance. The mixed solution was dropwise added to the mixture in the reactor over 230 minutes while carrying out bubbling with 5.2L/min of a nitrogen-air mixed gas having an oxygen concentration of 8 %, and stirring was carried out. After the completion of the addition, 1,500 g of water in which 55.68 g (146.5 mmol) of tetrasodium ethylenediamine tetraacetate was dissolved was added to the stirred mixture to terminate the reaction. An aqueous layer and an organic layer were separated. Then, the organic layer was washed with 1.0 N hydrochloric acid aqueous solution and then with pure water. The thus obtained solution was concentrated with an evaporator and then dried under a reduced pressure, to obtain 404.6 g of a bifunctional phenylene ether oligomer compound. The bifunctional phenylene ether oligomer compound had a number average molecular weight of 910, a weight average molecular weight of 1, 310 and a hydroxyl group equivalent of 455. Unreacted HMBP was 7.4 % and unreacted 2,6-dimethylphenol was 1.8 %. A peak corresponding to di-n-butylamine was detected in its $^1H$-NMR measurement. From the integration ratio of the peak (0.89 ppm) of its methyl group, it was confirmed that an amine adduct existed in an amount of 15 %.

(Comparative Example 3)

Example 4 was repeated except that 1.28 g (7.4 mmol) of N,N'-di-t-butylethylenediamine and 39.10 g (96.6 mmol) of n-butyldimethylamine were charged in the longitudinally long reactor in one lump, respectively, in place of the divided additions of these.

The amount of the thus obtained bifunctional phenylene ether oligomer compound was 408.6 g. The bifunctional phenylene ether oligomer compound had Mn of 870, Mw of 1, 380, Mw/Mn of 1.59 and a hydroxyl group equivalent of 440. Unreacted HMBP was 7.2 % and unreacted 2,6-dimethylphenol was 1.6 %. No peak corresponding to amine was detected in its $^1$H-NMR measurement and accordingly it was confirmed that no amine adduct generated.

Example 11

Comparative Example 2 was repeated except that, after the completion of the addition of the mixed solution, a maturation reaction was carried out for 120 minutes with continuing bubbling with the mixed gas.

The amount of the thus obtained bifunctional phenylene ether oligomer compound was 405.0 g. The bifunctional phenylene ether oligomer compound had Mn of 1, 220 and Mw of 3,500, Mw/Mn of 2.87. Dispersion was wide in the molecular weight distribution of GPC in comparison with the case where no maturation reaction was carried out and a new peak appeared in a high molecular region. The hydroxyl group equivalent was 720. Unreacted HMBP was 7.2 % and unreacted 2,6-dimethylphenol was 1.6 %. No peak corresponding to amine was detected in its $^1$H-NMR measurement and accordingly it was confirmed that no amine adduct generated.

[Table 1]

| | Bivalent/ monovalent phenols molar ratio | Divisional molar ratio (1) CuBr2 (2) DtBEDA (3) BDMA (4) DBA | Supply time of raw materials [min] | Maturation time [min] | Remaining monomer HMBP [wt%] | 2,6-X [wt%] |
|---|---|---|---|---|---|---|
| **Example 1** | 1 : 5 | (1) 25:75<br>(2) 25:75<br>(3) 25:75 | 230 | 0 | 1.5 | 0.1 |
| **Example 2** | 1 : 2 | (1) 40:60<br>(2) 40:60<br>(3) 40:60 | 230 | 0 | 1.3 | <0.1 |
| **Example 3** | 1 : 2 | (1) 40:60<br>(2) 40:60<br>(3) 40:60 | 230 | 0 | 1.1 | <0.1 |
| **Example 4** | 1 : 5 | (1) 50:50<br>(2) 50:50<br>(3) 50:50 | 230 | 0 | 0.5 | <0.1 |
| **Example 5** | 1 : 10 | (1) 60:40<br>(2) 60:40<br>(3) 60:40 | 230 | 0 | 0.2 | 0.2 |
| **Example 6** | 1 : 5 | (1) 50:50<br>(2) 50:50<br>(3) 50:50 | 230 | 0 | 0.9 | 0.1 |
| **Example 7** | 1 : 5 | (1) 50:50<br>(2) 50:50<br>(3) 50:50 | 95 | 0 | 0.8 | <0.1 |
| **Example 8** | 1 : 10 | (1) 55:45<br>(2) 60:40<br>(3) 40:60 | 230 | 0 | 0.3 | 0.1 |
| **Example 9** | 1 : 5 | (1) 100:0<br>(2) 50:50<br>(3) 50:50 | 230 | 0 | 4.3 | 0.9 |
| **Example 10** | 1 : 10 | (1) 100:0<br>(2) 60:40<br>(3) 60:40 | 230 | 0 | 3.9 | 0.7 |
| **Comparative Example 2** | 1 : 5 | (1) 100:0<br>(4) 100:0 | 230 | 0 | 7.4 | 1.8 |
| **Comparative Example 3** | 1 : 5 | (1) 50:50<br>(2) 100:0<br>(3) 100:0 | 230 | 0 | 7.2 | 1.6 |
| **Example 11** | 1 : 5 | (1) 100:0<br>(2) 50:50<br>(3) 50:50 | 230 | 120 | 2.1 | 0.1 |

DtBEDA: N,N'-di-t-butylethylenediamine, BDMA: n-butyldimethylamine, DBA:di-n-butylamine,
HMBP: 2,2',3,3',5,5'-hexamethyl-(1,1'-biphenyl)-4,4'-diol,
2,6-X: 2,6-dimethylphenol

[Table 1] (Continued)

| | Mn | Mw | Mw/Mn | OH equivalent [g/eq] | Amount of amine adduct [%] |
|---|---|---|---|---|---|
| Example 1 | 900 | 1,420 | 1.58 | 455 | Not Detected |
| Example 2 | 550 | 850 | 1.55 | 290 | Not Detected |
| Example 3 | 560 | 860 | 1.54 | 290 | Not Detected |
| Example 4 | 930 | 1,470 | 1.58 | 470 | Not Detected |
| Example 5 | 1,490 | 2,370 | 1.59 | 760 | Not Detected |
| Example 6 | 920 | 1,440 | 1.57 | 465 | Not Detected |
| Example 7 | 920 | 1,460 | 1.59 | 465 | Not Detected |
| Example 8 | 1,490 | 2,380 | 1.60 | 755 | Not Detected |
| Example 9 | 860 | 1,330 | 1.55 | 435 | Not Detected |
| Example 10 | 1,450 | 2,330 | 1.61 | 730 | Not Detected |
| Comparative Example 2 | 910 | 1,310 | 1.44 | 455 | 15 |
| Comparative Example 3 | 870 | 1,380 | 1.59 | 440 | Not Detected |
| Example 11 | 1,220 | 3,500 | 2.87 | 720 | Not Detected |

Mn: number average molecular weight, Mw: weight average molecular weight

Industrial Utilities

According to the production process of the present invention, it becomes possible to efficiently produce a bifunctional phenylene ether oligomer compound with a desired molecular weight and with no amine adduct, which oligomer compound has an extremely small remaining raw material phenol content and has a high quality, without any additional reaction such as a maturation reaction. Since the bifunctional phenylene ether oligomer compound obtained by the present invention has no amine adduct, its terminal phenolic hydroxyl group can be easily converted to a different functional group. In addition, since the remaining raw material phenol content is extremely small, the bifunctional phenylene ether oligomer compound can be applied to an electrical and electric material without impairing the features of a polyphenylene ether structure, which is the basic structure, such as heat resistance and dielectric characteristics.

**Claims**

1. A process for the production of a bifunctional phenylene ether oligomer compound having no amine adduct represented by the formula (1), which process comprises oxidatively polymerizing a bivalent phenol of the formula (2) and a monovalent phenol of the formula (3) in the presence of (a) a copper-containing catalyst and (b) an amine co-catalyst mixture consisting essentially of a tertiary amine and a secondary amine having a secondary alkyl group, a tertiary alkyl group or an aryl group, wherein 20% to 70% of the amine (b), based on the total amount thereof, is charged into a reactor in advance and the balance of 30 to 80% is added subsequently,

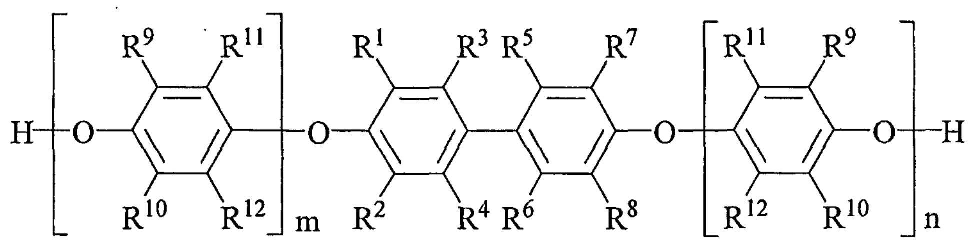

(1)

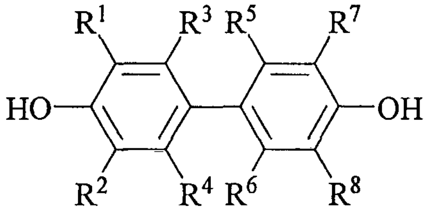

(2)

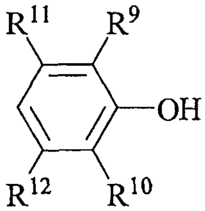

(3)

wherein $R^1$, $R^2$, $R^3$, $R^7$, $R^8$, $R^9$ and $R^{10}$ are the same or different and represent a halogen atom, an alkyl group having 6 or less carbon atoms or a phenyl group, $R^4$, $R^5$, $R^6$, $R^{11}$ and $R^{12}$ are the same or different and represent a hydrogen atom, a halogen atom, an alkyl group having 6 or less carbon atoms or a phenyl group, and each of m and n is an integer of from 0 to 25, provided that at least one of m and n is not 0.

2. A process according to claim 1, wherein 20 to 100 % of the catalyst (a), based on the total amount thereof, is charged in a reactor in advance and the balance of 0 to 80 % is added subsequently.

3. A process according to claim 1, wherein the monovalent phenol of the formula (3) is 2,6-dimethylphenol alone or a mixture of 2,6-dimethylphenol and 2,-3,6-trimethylphenol.

4. A process according to claim 1, wherein the molar ratio of the bivalent phenol of the formula (2) and the monovalent phenol of the formula (3) is 1:1 to 1:15.

**Patentansprüche**

1. Verfahren zur Herstellung einer bifunktionellen Phenylenetheroligomer-Verbindung, die kein Amin-Addukt aufweist, dargestellt durch die Formel (1), wobei das Verfahren ein oxidatives Polymerisieren eines bivalenten Phenols der Formel (2) und eines monovalenten Phenols der Formel (3) in der Anwesenheit (a) eines Kupfer-enthaltenden Katalysators und (b) einer Amin-Cokatalysator-Mischung, bestehend im Wesentlichen aus einem tertiären Amin und einem sekundären Amin mit einer sekundären Alkylgruppe, einer tertiären Alkylgruppe oder einer Arylgruppe, umfasst, wobei 20% bis 70% des Amins (b), basierend auf der Gesamtmenge davon, vorab in einen Reaktor gebracht wird und der Rest von 30 bis 80% darauffolgend zugegeben wird,

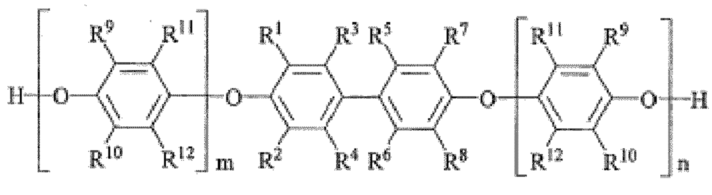

(1)

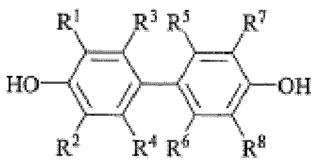

(2)

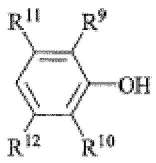

(3)

wobei $R^1$, $R^2$, $R^3$, $R^7$, $R^8$, $R^9$ und $R^{10}$ dieselben oder unterschiedlich sind und ein Halogenatom, eine Alkylgruppe mit sechs oder weniger Kohlenstoffatomen oder eine Phenylgruppe darstellen, $R^4$, $R^5$, $R^6$, $R^{11}$ und $R^{12}$ dieselben oder unterschiedlich sind und ein Wasserstoffatom, ein Halogenatom, eine Alkylgruppe mit sechs oder weniger Kohlenstoffatomen oder eine Phenylgruppe darstellen, und wobei jedes von m und n eine ganze Zahl von 0 bis 25 ist, vorausgesetzt, dass wenigstens eines von m und n nicht 0 ist.

**2.** Verfahren nach Anspruch 1, wobei 20 bis 100% des Katalysator (a), basierend auf der Gesamtmenge davon, in einen Reaktor vorab gebracht wird und der Rest von 0 bis 80% darauffolgend zugegeben wird.

**3.** Verfahren nach Anspruch 1, wobei das monovalente Phenol der Formel (3) 2,6-Dimethylphenol alleine oder eine Mischung von 2,6-Dimethylphenol und 2,3,6-Trimethylphenol ist.

**4.** Verfahren nach Anspruch 1, wobei das molare Verhältnis des bivalenten Phenols der Formel (2) und des monovalenten Phenols der Formel (3) 1:1 bis 1:15 ist.

## Revendications

**1.** Procédé de production d'un composé oligomère de phénylène éther bifonctionnel ne comprenant pas d'adduit d'amine représenté par la formule (1), comprenant la polymérisation par oxydation d'un phénol bivalent de formule (2) et d'un phénol monovalent de formule (3) en présence de (a) un catalyseur contenant du cuivre et (b) un mélange de co-catalyseurs de type amine essentiellement constitué d'une amine tertiaire et d'une amine secondaire comprenant un groupe alkyle secondaire, un groupe alkyle tertiaire ou un groupe aryle, dans lequel 20 % à 70 % de l'amine (b), par rapport à la quantité totale de celle-ci, sont chargés au préalable dans un réacteur et le complément de 30 à 80 % est ajouté par la suite,

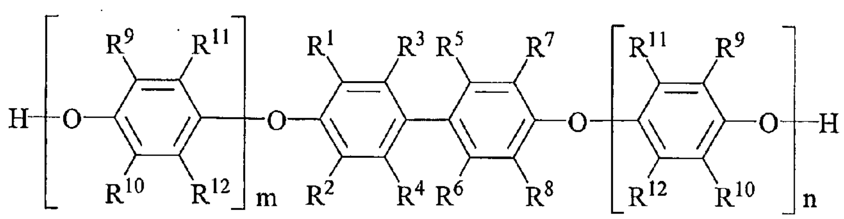

(1)

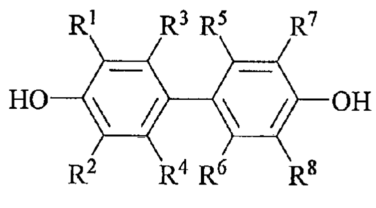

(2)

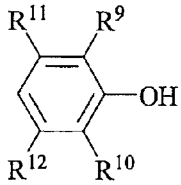

(3)

dans lesquelles $R^1$, $R^2$, $R^3$, $R^7$, $R^8$, $R^9$ et $R^{10}$ sont identiques ou différents et représentent un atome d'halogène, un groupe alkyle comportant 6 atomes de carbone ou moins ou un groupe phényle, $R^4$, $R^5$, $R^6$, $R^{11}$ et $R^{12}$ sont identiques ou différents et représentent un atome d'hydrogène, un atome d'halogène, un groupe alkyle comportant 6 atomes de carbone ou moins ou un groupe phényle, et chacun de m et n est un nombre entier de 0 à 25, à condition que l'un au moins de m et de n ne soit pas 0.

**2.** Procédé selon la revendication 1, dans lequel 20 % à 100 % du catalyseur (a), par rapport à la quantité totale de celui-ci, sont chargés au préalable dans un réacteur et le complément de 0 à 80 % est ajouté par la suite.

**3.** Procédé selon la revendication 1, dans lequel le phénol monovalent de formule (3) est le 2,6-diméthylphénol seul ou un mélange de 2,6-diméthylphénol et de 2,3,6-triméthylphénol.

**4.** Procédé selon la revendication 1, dans lequel le rapport molaire du phénol bivalent de formule (2) au phénol monovalent de formule (3) est de 1:1 à 1:15.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11021452 A
- JP 9291148 A
- JP 8011747 B
- JP 2003012796 A
- JP 52000897 A
- JP 62131022 A
- JP 5148357 A
- KR 20030032860
- KR 20030003107